# EUROPEAN PATENT APPLICATION

(11) **EP 2 194 380 A2**
(43) Date of publication of application: **09.06.2010**
(21) Application number: 10157725.2
(22) Date of filing: 05.07.2006
(51) Int. Cl.: G01N 33/53

(54) **Detection of a target antigen irrespective of the presence or absence of a corresponding therapeutic antibody**

(30) Priority: 06.07.2005 EP 05014618; 06.03.2006 EP 06004447
(62) Divisional of application: 06754671.3
(71) Applicant: F. Hoffmann-La Roche AG, 4070 Basel (CH)
(72) Inventor: The designation of the inventor has not yet been filed
(74) Representative: Vossius & Partner

(57) **Abstract**

The present invention relates to the field of therapeutic antibodies. The invention especially relates to a method of detecting the target antigen of a therapeutic antibody in a sample comprising the steps of a) providing the sample to be analyzed, b) incubating said sample with said therapeutic antibody under conditions appropriate for binding of said therapeutic antibody to said target antigen, whereby a target antigen-therapeutic antibody-complex is formed, and c) detecting the complex formed in (b).

## Description

The present invention relates to a method for detecting a target antigen irrespective of the presence or absence of a corresponding therapeutic antibody. It especially relates to the measurement of a target antigen in the presence of a corresponding therapeutic antibody. The present invention discloses a method of detecting the target antigen of a therapeutic antibody in a sample comprising the steps of a) providing the sample to be analyzed, b) incubating said sample with said therapeutic antibody under conditions appropriate for binding of said therapeutic antibody to said target antigen, whereby a target antigen-therapeutic antibody-complex is formed, and c) detecting the complex formed in (b). It also relates to the use of said method in the follow-up of a patient.

### Background of the Invention

Since the development of the first monoclonal antibodies by Köhler and Milstein in 1974 a lot of efforts have been dedicated to the development of antibodies which are appropriate for therapy in humans. The first monoclonal antibodies which became available had been developed in mice and rats. These antibodies when used for therapy of a human being caused unwanted side effects due to anti-rodent antibodies. A lot of efforts have been dedicated to the reduction or even elimination of such unwanted side effects.

In the past ten years an ever growing number of human monoclonal antibodies or humanized monoclonal antibodies have reached the market. Well-known examples include for example Herceptin^{®} and MabThera^{®} from Hoffmann-La Roche, Basel.

A quite significant number of human or humanized monoclonal antibodies is under investigation and needs to be studied in experimental animals, before entry into human can be considered for the first trial purposes.

Therapeutic monoclonal antibodies typically have to be used with serum levels ranging from about between 1 nanogram per ml to about 100 microgram per ml. The therapeutic antibody, thus at least at certain time-points during a treatment regimen, is present in quite high concentration, e.g. in a concentration which is as high or even higher as the concentration of the corresponding target antigen.

A correct measurement of the target antigen itself is considered very important especially in the follow-up of patients after therapy and especially after therapy with a corresponding therapeutic antibody. Since during the course of a treatment regimen the concentration of a therapeutic antibody will vary to a large extent any interference of such therapeutic antibody in an assay set up for the measurement of its corresponding target antigen may and most likely will lead to false measurements for said target antigen.

The level of a target antigen may be detected by any appropriate method. In clinical routine such methods in most cases will employ antibodies to the target antigen, the so-called immure assays. A high and/or variable concentration of a therapeutic antibody may interfere in the immuno assay used to measure the level of its target antigen.

As the skilled artisan will readily appreciate, it is not possible, or at least not an easy task, to use the therapeutic antibody itself in the detection of its corresponding target antigen. The same difficulty will frequently be encountered if the therapeutic antibody and at least one of the antibodies used in an immuno assay bind to the same epitope of a target antigen. Whereas this fact is well-known and generally accepted, it has now been surprisingly found that an immuno assay for detection of a target antigen may also be compromised by the presence or absence of a therapeutic antibody even if the therapeutic antibody binds to an epitope not bound by the antibody or the antibodies used in an immuno assay for the corresponding target antigen.

Jilani et al. (Jilani, I., et al., Blood 1032 (2003) 3514-3520) reported that to detect rituximab on the cell surface, antibodies were used that specifically detected the mouse sequence in rituximab and did not cross-react with the human Ig. In WO 03/024993 a method of detecting and monitoring a therapeutic antibody:antigen complex, soluble antigen, free therapeutic antibody and soluble total therapeutic antibody is reported.

It was a task of the present invention to investigate whether methods of detecting a target antigen of a therapeutic antibody in a sample can be improved. Such method should yield a true and correct value for the concentration of the target antigen, no matter whether the corresponding therapeutic antibody is present or not and should be of use in consecutive measurements, e.g. in the follow-up of patients. This task has been accomplished by the invention as described below and in the examples section and as claimed in the appending claims.

### Summary of the Invention

The invention comprises a method of detecting the target antigen of a therapeutic antibody in a sample comprising the steps
a) providing the sample to be analyzed,
b) incubating said sample with said therapeutic antibody under conditions appropriate for binding of said therapeutic antibody to said target antigen, whereby a target antigen-therapeutic antibody-complex is formed with the total target antigen being complexed by the therapeutic antibody, and
c) detecting the complex formed in b).

### Detailed Description of the Invention

In a first embodiment the present invention relates to a method of detecting the target antigen of a therapeutic antibody in a sample comprising the steps of a) providing the sample to be analyzed, b) incubating said sample with said therapeutic antibody under conditions appropriate for binding of said therapeutic antibody to said target antigen, whereby a target antigen-therapeutic antibody-complex is formed, and c) detecting the complex formed in b).

The term "target antigen" relates to a biomolecule which is bound by its corresponding therapeutic antibody. By way of example, the target antigen of a therapeutic antibody to HER2 (= ErbB2 or p 185 *^{neu}*), like Herceptin^{®} or Omnitarg^{®}, is HER2, of a therapeutic antibody to CD52, like Campath^{®}, is CD52, of a therapeutic antibody to EGFr, like Erbitux^{®}, is EGFr, of a therapeutic antibody to CD33, like Mylotarg^{®}, is CD33, of a therapeutic antibody to Tag-72, like C3ncoScint^{®}, is Tag-72, of a therapeutic antibody to 17-1A, like Panorex^{®}, is 17-1A, of a therapeutic antibody to CD20, like Rituxan^{®}), MabThera^{®}, or Zevalin^{®}, is CD20, and of a therapeutic antibody to CD25, like Zenapax^{®}, is CD25. The target antigen may either be a soluble, i.e. secreted or shed, target antigen or a (cell-)membrane bound target antigen.

In another embodiment said target antigen-therapeutic antibody-complex formed in step b) is formed with the total target antigen being complexed by the therapeutic antibody.

The term "soluble target antigen" as used within this application denotes the soluble form, i.e. secreted or shed form, of a membrane-bound target antigen of a therapeutic antibody. Therapeutic antibodies mostly are directed against cell surface antigens, e.g. of cancer cells, to which they bind. Beside the membrane-bound variant of a cell surface antigen, secreted or shed, i.e. soluble, variants of such an antigen can be produced by cells. The soluble target antigen can be found in body fluids of an affected individual. The "soluble target antigen" is the secreted or shed variant of a membrane-bound antigen, whereby the soluble variant possesses the same amino acid sequence and the same secondary structure as at least a part of the extracellular domain of the membrane-bound antigen, thus allowing an antibody directed against the extracellular domain of a (membrane-bound) target antigen also to bind the soluble target antigen.

In another embodiment said target antigen is a soluble target antigen.

The term "epitope" as used within this application denotes a protein determinant capable of specific binding to an antibody. Epitopes usually consist of chemically active surface groupings of molecules such as amino acids or sugar side chains and usually have specific three dimensional structural characteristics, as well as specific charge characteristics. Conformational and non-conformational epitopes are distinguished in that the binding to the former but not the latter is lost in the presence of denaturing solvents. Depending on the size of the antigen to which the epitope belongs, more than one epitope per antigen may be available resulting likewise in the possibility of more than one antibody binding site (=epitopes) per antigen.

A "sample" according to the present invention may be any tissue or liquid sample removed from the experimental animal, preferably from a mammal. Preferably the sample will be a liquid sample like saliva, urine, whole blood, plasma or serum. Preferably the sample will be whole blood, plasma or serum. Preferably the sample is a cell-free sample, i.e. a sample containing no cells bearing membrane-bound target antigen.

Conditions which are appropriate for binding of a target antigen to its corresponding "therapeutic antibody" are well-known to the skilled artisan or can easily be determined. Under these conditions the therapeutic antibody binds to the target antigen, either the membrane-bound or soluble variant, and an immunological complex between the target antigen and the therapeutic antibody is formed, resulting in a target antigen-therapeutic antibody-complex. This complex can be detected by any appropriate means.

In a preferred embodiment a target antigen-therapeutic antibody-complex is detected by aid of an immuno assay. The immuno assay used preferably is a heterogeneous immuno assay. It is also preferred that the detection of the target antigen-therapeutic antibody-complex is accomplished by aid of a competitive immuno assay or by aid of a so-called sandwich immuno assay.

The skilled artisan will have no problem in setting up an immuno assay, which is capable of detecting the target antigen as present in the target antigen-therapeutic antibody-complex. By way of example such detection may be performed in a sandwich type immuno assay wherein an antibody is used as a capture antibody, which is binding to the target antigen at an epitope which does not overlap with the epitope of the therapeutic antibody. For detection of the target antigen-therapeutic antibody-complex it is preferred to use a second or detection antibody to the target antigen which binds to an epitope neither recognized by the therapeutic antibody nor by the capture antibody.

Preferably a detection antibody capable of forming a detection antibody-target antigen-therapeutic antibody-complex sandwich is used. Said second or detection antibody preferably is labeled in such a manner that direct or indirect detection is facilitated.

For direct detection the labeling group can be selected from any known detectable marker groups, such as dyes, luminescent labeling groups, such as chemiluminescent groups, *e.g*., acridinium esters or dioxetanes, or fluorescent dyes, *e.g*., fluorescein, coumarin, rhodamine, oxazine, resorufin, cyanine and derivatives thereof. Other examples of labeling groups are luminescent metal complexes, such as ruthenium or europium complexes, enzymes, *e.g*., as used for ELISA or for CEDIA (Cloned Enzyme Donor Immunoassay, *e.g*., EP 0 061 888), and radioisotopes.

Indirect detection systems comprise, for example, that the detection reagent, e.g. the detection antibody, is labeled with a first partner of a bioaffine binding pair. Examples of suitable binding pairs are hapten or antigen/antibody, biotin or biotin analogues such as aminobiotin, iminobiotin or desthiobiotin/avidin or streptavidin, sugar/lectin, nucleic acid or nucleic acid analogue/complementary nucleic acid, and receptor/ligand, *e.g*., steroid hormone receptor/steroid hormone. Preferred first binding pair members comprise hapten, antigen and hormone. Especially preferred are hastens like digoxin, digoxigenin and biotin and analogues thereof. The second partner of such binding pair, e.g. an antibody, streptavidin, etc., usually is labeled to allow for direct detection, e.g., by the labels as mentioned above.

Immunoassays are well known to the skilled artisan. Methods for carrying out such assays as well as practical applications and procedures are summarized in related textbooks. Examples of related textbooks are Tijssen, P., Preparation of enzyme-antibody or other enzyme-macromolecule conjugates, in: Practice and theory of enzyme immunoassays, Burden, R.H. and v. Knippenberg, P.H. (eds.), Elsevier, Amsterdam (1990) pp. 221-278; and various volumes of Methods in Enzymology, Colowick, S.P. and Caplan, N.O. (eds.), Academic Press, dealing with immunological detection methods, especially volumes 70, 73, 74, 84, 92 and 121.

In all the above immunological detection methods reagent conditions are chosen which allow for binding of the reagents employed, e.g. for binding of an antibody to its corresponding antigen. The target antigen-therapeutic antibody-complex detected according to the present invention is correlated by state of the art procedures to the corresponding concentration of the target antigen, either in membrane-bound form or soluble form.

The antibody or antibodies binding to the target antigen at an epitope not recognized by the therapeutic antibody may be a polyclonal antibody, a monoclonal antibody, fragments of such antibodies, as well as genetic constructs comprising the binding domain of such antibody. Appropriate antibody fragments can also be used. Antibodies as well as antibody fragments are generated by state of the art procedures, e.g., as described in Tijssen (Tijssen, P., Practice and theory of enzyme immunoassays 11 (1990), the whole book, especially pp. 43-78, Elsevier, Amsterdam).

The term "therapeutic antibody" relates to any antibody preparation which is intended for use in a human being. Preferably such therapeutic antibody will be a monoclonal antibody. Further preferred such monoclonal antibody will be obtained from a great ape or be a human monoclonal antibody or a humanized antibody. Preferably, it will be a human monoclonal antibody. Also preferred such therapeutic monoclonal antibody will be a humanized monoclonal antibody.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to polyclonal antibody preparations, which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Köhler, G., et al., Nature 256 (1975) 495-497, or may be made by recombinant DNA methods (see, e.g., US 4,816,567). The "monoclonal antibodies" may also be isolated from phage antibody libraries using the techniques described in Clackson, T., et al., Nature, 352 (1991) 624-628 and Marks, J. D., et al., J. Mol. Biol. 222 (1991) 581-597, for example.

"Humanized" forms of non-human (e.g. rodent) antibodies are chimaeric antibodies that contain partial sequences derived from non-human immunoglobulin and from a human immunoglobulin. For the most part, humanized antibodies are derived from a human immunoglobulin (recipient antibody) in which residues from a hypervariable region of the recipient are replaced by residues from a hypervariable region of a non-human species (donor antibody) such as mouse, rat, rabbit or non-human primate having the desired specificity and affinity. In some instances, framework region (FR) residues of the human immunoglobulin are replaced by corresponding non-human residues. Furthermore, humanized antibodies may comprise further modifications, e.g., amino acid residues that are not found in the recipient antibody or in the donor antibody. Such modifications result in variants of such recipient or donor antibody which are homologous but not identical to the corresponding parent sequence. These modifications are made to further refine antibody performance. In general, the humanized antibody will comprise substantially all of at least one, and typically two, variable domains, in which all or substantially all of the hypervariable loops correspond to those of a non-human donor antibody and all or substantially all of the FRs are those of a human recipient antibody. The humanized antibody optionally also will comprise at least a portion of an immunoglobulin constant region (Fc), typically that of a human immunoglobulin.

Methods for humanizing non-human antibodies have been described in the art. Preferably, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones, P. T., et al., Nature 321 (1986) 522-525; Riechmann, L., et al., Nature 332 (1988) 323-327; Verhoeyen, M., et al., Science 239 (1988) 1534-1536; and Presta, L. G., Curr. Op. Struct. Biol. 2 (1992) 593-596), by substituting hypervariable region sequences for the corresponding sequences of a human antibody. Accordingly, such "humanized" antibodies are chimaeric antibodies (US 4,816,567), wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework region (FR) for the humanized antibody (Sims, M. J., et al., J. Immunol. 151 (1993) 2296-2308; Chothia, C., et al., J. Mol. Biol. 196 (1987) 901-917). Another method uses a particular framework region derived from the consensus sequence of all human antibodies of a particular subgroup of light or heavy chains. The same framework may be used for several different humanized antibodies (Carter, P., et al., Proc. Natl. Acad. Sci. USA 89 (1992) 4285-4289; Presta, L. G., et al., J. Immune 151 (1993) 2623-2632).

Immunoglobulins can be generated against, e.g., human, mouse, or rat polypeptides. Immunoglobulins, either polyclonal or monoclonal, specifically recognizing the target antigen are encompassed by the invention. Such immunoglobulins are raised using standard immunological techniques known to a person skilled in the art. Immunoglobulins may be polyclonal or monoclonal or may be produced recombinantly such as for a humanized antibody. The determination if an antibody is not binding to the same epitope as a known therapeutic antibody can easily be determined in a competitive test system.

Possible epitope overlapping of two antibodies binding to the same target antigen can be detected with the help of a competitive test system. For this purpose, for example with the help of an enzyme immunoassay, there is tested the extent to which the new antibody competes with the known antibody for the binding to an immobilized target antigen. For this purpose, an appropriately immobilized target antigen is incubated with the known antibody in labeled form and an excess of the antibody in question. By detection of the bound labeling there can easily be ascertained the extent to which the antibody in question can displace the known antibody from the binding site (= epitope). If there is a displacement of not more than 20%, preferably of not more than 10%, at the same concentration or at higher concentrations, preferably in the case of 10⁵-fold excess of the antibody in question, referred to the known antibody, then no epitope overlapping is present.

Well known examples of humanized therapeutic antibodies are the so-called anti-ErbB2 antibodies including huMAb4D5-1, huMAb4D5-2, huMAb4D5-3, huMAb4D5-4, huMAb4D5-5, huMAb4D5-6, huMAb4D5-7 and huMAb4D5-8 (HERCEPTIN^{®}) as described in Table 3 of US 5,821,337 expressly incorporated herein by reference; as well as humanized 520C9 (described in WO 93/21319) and humanized 2C4 antibodies as described in PCT/US 03/21590.

Preferably the therapeutic antibody used in a method according to the present invention is selected from the group consisting of Campath^{®}, Erbitux^{®}, Herceptin^{®}, h4abThera^{®}, Mylotarcg^{®}, OncoScint^{®}, Omnitarg^{®}, Panorex^{®}, Rituxan^{®}, Zevalin^{®} and Zenapax^{®}, preferably of the group consisting of Herceptin^{®}, MabThera^{®}, Omnitarg^{®} and Zenapax^{®}.

Zevalin^{®} is the trade name for the therapeutic antibody also known as ibritumomab. This therapeutic antibody is based on monoclonal antibody which binds to CD20 and is approved by the FDA for the treatment of B-cell non-Hodgkins lymphoma.

Rituxan^{®} is the trade name for the therapeutic antibody also known as rituximab. This therapeutic antibody is based on monoclonal antibody which binds to CD20 and is approved by the FDA for the treatment of B-cell non-Hodgkins lymphoma. Panorex^{®} is the trade name for the therapeutic antibody also known as edrecolomab. This therapeutic antibody binds to the 17-1A antigen (Ep-CAM) and has been approved for the treatment of colorectal cancer.

OncoScint^{®} is the trade name for the therapeutic antibody also known as satumomab. This therapeutic antibody binds to the pancarcinoma antigen Tag-72 and has been approved for the treatment of colon and ovarian carcinoma.

Mylotarg^{®} is the trade name for the therapeutic antibody also known as gemtuzmab. This therapeutic antibody is based on monoclonal antibody which binds to CD33 and is approved by the FDA for the treatment of myeloid leukemia.

Erbitux^{®} is the trade name for the therapeutic antibody also known as cetuximab. This therapeutic antibody binds to epidermal growth factor receptor (EGFr) and has been approved for colorectal cancer.

Campath^{®} is the trade name for the therapeutic antibody also known as alemtuzumab. This therapeutic antibody is based on monoclonal antibody which binds to CD52 and is approved by the FDA for the treatment of chronic lymphocytic leukemia.

Herceptin^{®} is the trade name for the therapeutic antibody also known as trastuzumab. This therapeutic antibody is based on monoclonal antibody 4D5. It binds to HER2. HER2 is also known as ErbB2 or p 185 *^{neu}*. Herceptin^{®} has been shown to have a positive effect on survival of HER2-positive patients with breast cancer (De Laurentiis, M., et al., Ann. Oncol. 16 (2005) iv7-iv13).

MabThera^{®} is the trade name for the therapeutic antibody also known as rituximab. This therapeutic antibody is based on monoclonal antibody which binds to CD20. MabThera^{®} has been shown to have a positive effect on survival of patients suffering from indolent and aggressive non-Hodgkin's lymphoma (Di Bella, N., et al., Cancer 103 (2005) 978-984).

Omnitarg^{®} is the trade name for a novel therapeutic antibody pertuzumab binding to HER2. It is based on monoclonal antibody 2C4. 2C4 and 4D5 bind to different epitopes on HER2. Omnitarg^{®} is currently subject to clinical trials. It is hoped that it will have a positive effect on survival for HER2-overexpression-negative patients with breast cancer (Badache, A., et al., Cancer Cell 5 (2004) 299-301).

Zenapax^{®} is the trade name for a therapeutic antibody binding to the interleukin-2 receptor. It is associated with decreased rejection and improved patient survival in renal transplant recipients (Morris, J.A., et aL, Clin. Transplant. 19 (2005) 340-345).

It is also preferred that the target antigen detected in a method according to the present invention is selected from the group consisting of HER2, interleukin-2 receptor, IGF-1R, EGFr, Tag-72, 17-1A, CD52, CD25, CD33 or CD20, preferably of the group consisting of HER2, interleukin-2 receptor, IGF-1R, and CD20, preferably of the group consisting of HER2, interleukin-2 receptor, or CD20.

As mentioned above it was found by the inventors of the present invention that not only therapeutic antibodies which bind to an epitope which is also bound by an antibody used in an assay for detecting the corresponding target antigen will interfere with such assay. Interference may as well occur and has been observed in assay methods using antibodies to non-overlapping epitopes on the target antigen. The specific observations presented in the Examples section have been made with assays for the HER2 antigen and two different therapeutic antibodies binding this target antigen. However, it has to be expected that the same will hold true in the exact quantification of other target antigens. The examples demonstrate that for both the different antibodies to HER2, i.e. Herceptin^{®} and Cmnitarg^{®}, the method according to the present invention can be applied and the target antigen HER2 can be reliably measured independent of the presence or absence of the corresponding therapeutic antibody in the sample investigated,

In one embodiment the therapeutic antibody is Herceptin^{®}, the capture antibody is Omnitarg^{®}, and the detection antibody is the anti-ErbB2 antibody 7C2.

In another embodiment the therapeutic antibody is Omnitarg^{®}, the capture antibody is Herceptin^{®}, and the detection antibody is the anti-ErbB2 antibody 7C2.

Herceptin^{®}, Omnitarg^{®}, and the anti-ErbB2 antibody 7C2 bind to different epitopes of the HER2 antigen. The anti-ErbB2 antibody 7C2 e.g. recognizes an epitope in the region of the N-terminus of ErbB2 (see e.g. WO 98/17797).

In one embodiment in the method of the invention the target antigen-therapeutic antibody-complex formed in step b) is formed whereby the total target antigen in the sample is being complexed by the therapeutic antibody.

The invention comprises a method of detecting the target antigen of a therapeutic antibody in a sample comprising the steps
a) providing the sample to be analyzed,
b) incubating said sample with said therapeutic antibody under conditions appropriate for binding of said therapeutic antibody to said target antigen, whereby a target antigen-therapeutic antibody-complex is formed with the total target antigen being complexed by the therapeutic antibody, and
c) detecting the complex formed in b).

The reliable measurement of a target antigen will be of significant advantage for any sample comprising an interfering therapeutic antibody. In a preferred embodiment the present invention therefore relates to a method of detecting the target antigen of a therapeutic antibody in a sample wherein said therapeutic antibody is present comprising the steps of a) providing the sample to be analyzed, b) incubating said sample with said therapeutic antibody under conditions appropriate for binding of said therapeutic antibody to said target antigen, whereby a target antigen-therapeutic antibody-complex is formed, and c) detecting the complex formed in b).

The method according to the present invention will also be of great use in the follow-up of patients which receive a therapeutic antibody. In a preferred embodiment the present invention therefore relates to a method of detecting the target antigen of a therapeutic antibody in a sample obtained from a patient under therapy with said therapeutic antibody comprising the steps of a) providing the sample to be analyzed, b) incubating said sample with said therapeutic antibody under conditions appropriate for binding of said therapeutic antibody to said target antigen, whereby a target antigen-therapeutic antibody-complex is formed, and c) detecting the complex formed in b).

The correct determination of the corresponding target antigen will in a preferred embodiment be useful to assess the efficacy of therapy. In a further preferred embodiment the method according to this invention will be of help in assessing a relapse to the underlying disease.

In general the clinician may derive valuable information in the follow-up of patients under therapy with a therapeutic antibody by use of the method presented herein. The present invention thus also relates to the use of a method as presented above in the follow-up of patients.

Without wishing to be bound to the suggested explanation it may be that the binding of a therapeutic antibody to its target antigen has, e.g., some influence on the conformation of other epitopes and/or the binding properties of other antibodies to these epitopes as present in a target antigen-therapeutic antibody-complex. Preferably the method according to the present invention is used even if the epitopes on a target antigen for the antibodies as used in an immuno assay and for the therapeutic antibody, respectively, do not overlap.

In one embodiment the method of the invention is performed on a chip.

A "chip" is a solid, non porous material, such as metal, glass or plastics. The material may optionally be coated, entirely or in certain areas. On the surface of the material any array of spots may be/is present, either visible or in coordinates. On each spot a defined polypeptide, with or without linker or spacer to the surface of the material, may be immobilized. Preferably the immobilized polypeptides are at least fragments of antibodies which are capable of binding the target antigen.

The following examples and figures are provided to aid the understanding of the present invention, the true scope of which is set forth in the appended claims. It is understood that modifications can be made in the procedures set forth without departing from the spirit of the invention.

### Description of the Figures

- **Figure 1**: Interference of two different therapeutic antibodies in HER2- detection using the commercial assay sold by Oncogene Sciences.
- **Figure 2**: Interference of two different therapeutic antibodies in HER2- detection using the commercial assay sold by Bender.
- **Figure 3**: Determination of the maximum of interference for the therapeutic antibody Omnitarg^{®}.
- **Figure 4**: Determination of the maximum of interference for the therapeutic antibody Herceptin^{®}.

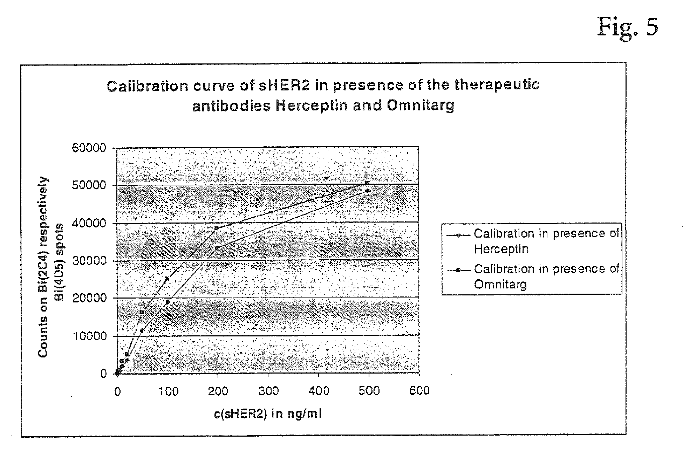

- **Figure 5**: Calibration curves of HER2-detection using assays devoid of interference by Omnitarg^{®} and Herceptin^{®}, respectively.

### Abbreviation

- Bi: biotin
- EDTA: ethylenediaminetetraaceticacid
- ELISA: enzyme-linked immunosorbent assay
- Fcy (=Fcγ): Fc gamma-fragment of an immunoglobulin
- DIG (Dig): digoxigenin
- hu, H: human
- IgG: immunoglobulin G
- n. d.: not defined
- NSCLC: non-small cell lung cancer
- OLD: optical density
- MAK (=Mab): monoclonal antibody
- PAK (=Pab): polyclonal antibody
- R: cow (Rind)
- RPLA: Bovine-Plasmaalbumin
- RT: room temperature
- TAPS: N-tris(hydroxymethyl)methyl-3-aminopropanesulfonic acid
- VEGF: vascular endothelial growth factor

### Example 1

### Commercial Assays for HER2

### a) HER-2/neu ELISA manufactured by Oncogene Science/Bayer Health Care LLC (Cat. No. DAKO Cytomation EL5011)

Incubations and washing steps were performed according to the instructions given by the manufacturer. Standards of 35, 25, 15, 7.5, 2.5, and 0 ng/ml HER2 were spiked with 500, 250, 0 µg/ml Herceptin^{®} or they were spiked with 500, 250, 0 µg/ml Onnitarg^{®}, respectively. These samples were incubated on the coated microtiter-plate. After adding and incubating the detection antibody, the substrate was added and the absorbance was read at 492 nm. Results are given in Table 1.

**Table 1**

| **Optical densities (ODs) as measured with and without interfering therapeutic antibody in the sample** | | | | |
|---|---|---|---|---|
| **standard spiked with** | **c(HER2) in ng/ml** | **OD 492 nm** | **OD corrected with blank** | **difference to unspiked standards** |
| Herceptin^{®} 250 µg/ml | 34.3 | 2.246 | 1.833 | 3.5% |
| Herceptin^{®} 250 µg/ml | 24.5 | 1.829 | 1.416 | 0.1% |
| Herceptin^{®} 250 µg/ml | 14.7 | 1.263 | 0.850 | 7.8% |
| Herceptin^{®} 250 µg/ml | 7.4 | 0.853 | 0.440 | -3.0% |
| Herceptin^{®} 250 µg/ml | 2.5 | 0.580 | 0.167 | -25.6% |
| Herceptin^{®} 250 µg/ml | 0 | 0.396 | -0.017 | n.d. |
| Herceptin^{®} 500 µg/ml | 33.6 | 2.184 | 1,771 | 6.8% |
| Herceptin^{®} 500 µg/ml | 24 | 1.726 | 1.313 | 7.3% |
| Herceptin^{®} 500 µg/ml | 14.4 | 1.204 | 0.791 | 14.2% |
| Herceptin^{®} 500 µg/ml | 7.2 | 0.798 | 0.385 | 9.8% |
| Herceptin^{®} 500 µg/ml | 2.4 | 0.514 | 0.101 | 24.1% |
| Herceptin^{®} 500 µg/ml | 0 | 0.395 | -0.018 | n.d. |
| Omnitarg^{®} 250 µg/ml | 34.3 | 2.056 | 1.643 | 13.5% |
| Omnitarg^{®} 250 µg/ml | 24.5 | 1.591 | 1.178 | 16.9% |
| Omnitarg^{®} 250 µg/ml | 14.7 | 1.106 | 0.693 | 24.8% |
| Omnitarg^{®} 250 µg/ml | 7.4 | 0.762 | 0.349 | 18.3% |
| Omnitarg^{®} 250 µg/ml | 2.5 | 0.524 | 0.111 | 16.5% |
| Omnitarg^{®} 250 µg/ml | 0 | 0.401 | -0.012 | n.d. |
| Omnitarg^{®} 500 µg/ml | 33.6 | 1.939 | 1.526 | -6.0% |
| Omnitarg^{®} 500 µg/ml | 24 | 1.544 | 1.131 | -15.7% |
| Omnitarg^{®} 500 µg/ml | 14.4 | 1.128 | 0.715 | -34.3% |
| Omnitarg^{®} 500 µg/ml | 7.2 | 0.754 | 0.341 | -38.1% |
| Omnitarg^{®} 500 µg/ml | 2.4 | 0.493 | 0.080 | -19.4% |
| Omnitarg^{®} 500 µg/ml | 0 | 0.395 | -0.018 | n.d. |
| unspiked | 35 | 2.313 | 1.900 | |
| unspiked | 25 | 1.830 | 1.417 | |
| unspiked | 15 | 1.335 | 0.922 | |
| unspiked | 7.5 | 0.840 | 0.427 | |
| unspiked | 2.5 | 0.546 | 0.133 | |
| unspiked (blank) | 0 | 0.413 | 0.000 | |

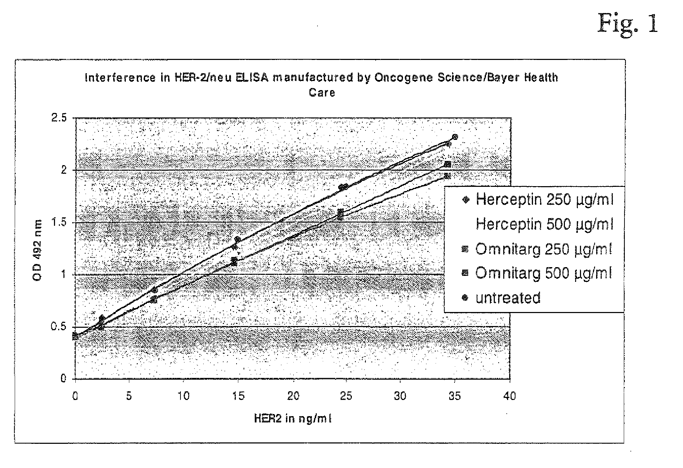

The difference in recovery of HER2 antigen due to spiking the therapeutic antibody Omnitarg^{®} is up to 38.1% and due to the spiking of Herceptin^{®} is up to 25.6%, respectively. The interference by Omnitarg^{®} and Herceptin^{®} is also evident from Fig. 1.

### b) sp185 ^{HER-2}Module Set manufactured by Bender MedSystems (Cat. No. Biozol BMS207MST)

Incubations and washing steps were performed according to the instructions given by the manufacturer. Standards of 20, 10, 5, 2.5, and 0 ng/ml HER2 were spiked with 500, 250, 0 µg/ml Herceptin^{®} or they were spiked with 500, 250, 0 µg/ml Omnitarg^{®}, respectively. These samples were incubated on the coated microtiter-plate. After adding and incubating the detection antibody, the substrate was added and the absorbance was read at 405 nm. Results are given in Table 2.

**Table 2**

| **Optical densities (ODs) as measured with and without interfering therapeutic antibody in the sample** | | | | |
|---|---|---|---|---|
| **standard spiked with** | **c(HER2) in ng/ml** | **OD 405 nm** | **OD corrected with blank** | **difference to unspiked standards** |
| Herceptin^{®} 250 µg/ml | 20 | 0.043 | 0.001 | 98.6% |
| Herceptin^{®} 250 µg/ml | 10 | 0.043 | 0.001 | 97.1% |
| Herceptin^{®} 250 µg/ml | 5 | 0.043 | 0.001 | 95.0% |
| Herceptin^{®} 250 µg/ml | 2.5 | 0.044 | 0.002, | 77.8% |
| Herceptin^{®} 250 µg/ml | 0 | 0.044 | 0.002 | n.d. |
| Herceptin^{®} 500 µg/ml | 20 | 0.044 | 0.002 | 97.1% |
| Herceptin^{®} 500 µg/ml | 10 | 0.044 | 0.002 | 94.3% |
| Herceptin^{®} 500 µg/ml | 5 | 0.043 | 0.001 | 95.0% |
| Herceptin^{®} 500 µg/ml | 2.5 | 0.044 | 0.002 | 77.8% |
| Herceptin^{®} 500 µg/ml | 0 | 0.044 | 0.002 | n.d. |
| Omnitarg^{®} 250 µg/ml | 20 | 0.105 | 0.063 | 10.0% |
| Omnitarg^{®} 250 µg/ml | 10 | 0.071 | 0.029 | 17.1% |
| Omnitarg^{®} 250 µg/ml | 5 | 0.058 | 0.016 | 20.0% |
| Omnitarg^{®} 250 µg/ml | 2.5 | 0.051 | 0.009 | 0.0% |
| Omnitarg^{®} 250 µg/ml | 0 | 0.043 | 0.001 | n.d. |
| Omnitarg^{®} 500 µg/ml | 20 | 0.103 | 0.061 | 12.9% |
| Omnitarg^{®} 500 µg/ml | 10 | 0.067 | 0.025 | 28.6% |
| Omnitarg^{®} 500 µg/ml | 5 | 0.059 | 0.017 | 1.5.0% |
| Omnitarg^{®} 500 µg/ml | 2.5 | 0.050 | 0.008 | 11.1% |
| Omnitarg^{®} 500 µg/ml | 0 | 0.044 | 0.002 | n.d. |
| unspiked | 20 | 0.112 | 0.070 | |
| unspiked | 10 | 0.077 | 0.035 | |
| unspiked | 5 | 0.062 | 0.020 | |
| unspiked | 2.5 | 0.051 | 0.009 | |
| unspiked (blank) | 0 | 0.042 | 0.000 | |

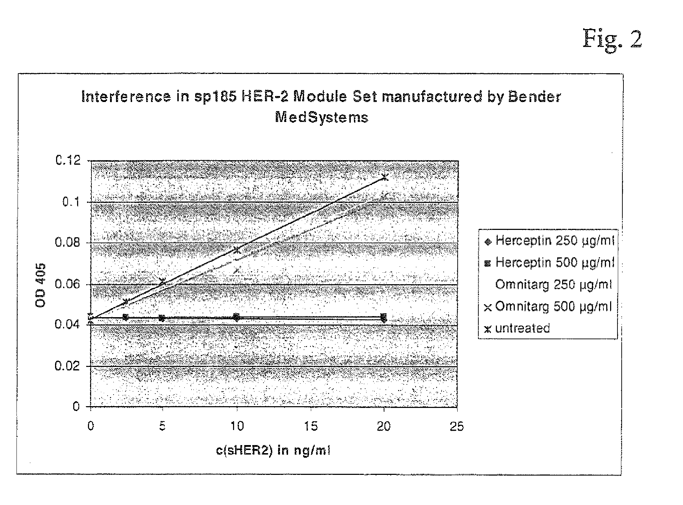

The difference in recovery of HER2 antigen due to spiking with the therapeutic antibody Herceptin^{®} is up to 98.6% and due to Omnitarg^{®} up to 28.6%. This is also depicted in Fig. 2.

### Example 2

### Assay for detection of HER2 in the presence of Omnitare^{®}

The assay was performed on streptavidin coated polystyrene chips. The antibody to HER2 was applied to the chip as lines of approximately ten 250 pL droplets. MAK<Her2>H-4D5-IgG-Bi (=biotinylated monoclonal antibody from clone 4D5 against HER2) was used as a capture antibody to establish an assays without interference by Omnitarg^{®}. The concentration of the biotinylated antibodies was 100 µg/ml. The chips were stored at 4°C.

MAK<Herz>M-7C2-IgG-Dig (digoxigenylated monoclonal antibody from clone 7C2) was used as conjugate antibody. The stock solution of this conjugate was stored at -20°C.

The HER2 standard protein sp185 (HER-2) (sHER2), was a commercially available product (Biozol #BMS207MST S), and has been stored at -20°C in a concentration of 1000 ng/ml.

As the basic sample and conjugate buffer a phosphate buffered saline (50mM sodium dihydrogenphosphate-monohydrate, 150mM NaCl at pH 7), comprising sodium azide (0.09% Na-azide) as a preservative, and further additives (0.035% EDTA, 0.05% Tween 20^{®}, 2.00% RPLA4 (Roche Nr. 1726544001), 0.10% PAK<->R-IgG (Roche Nr. 1108750001), 100µg/ml mouse-MAK-33-IgG-Poly (Roche Nr. 1939661001)) was used. The sample and conjugate buffer has been filtered (0.2µm pore size) and stored at 4°C prior to use.

The stock solution of the therapeutic antibody Omnitarg^{®} rhuMAb 2C4 G186 CP R9805AX - produced by Genentech, Inc. - had a concentration of 25 mg/ml and was stored at 4°C.

As detection antibody MAK<Dig>M19-11-IgG conjugated to 110 nm fluorescent labeled latex particles has been used. The detection antibody conjugate has been stored at 4°C.

As the detection buffer a 50 mM TAPS and 1M NaCl buffer at pH 8.5, comprising sodium azide (0.09% Na-azide) as a preservative, and further additives (0.05% Tween 20^{®}, 0.50% RPLA4 (Roche Nr. 1726544001), 10µg/ml anouse-MAK-33-IgG-Poly (Roche Nr. 1939661001)) was used. The detection buffer has been filtered (0,2µm pore size) and stored at 4°C prior to use.

The washing buffer was a 10mM Tris/HCl buffer with pH 8.0 comprising 0.001% Oxypyrion, 0.001% MIT, and 0.01% Thesit. The washing buffer has been filtered (0.2µm pore size) and stored at 4°C prior to use.

### a) Detection of a potential interference by Omnitarg^{®}

The assay was performed at room temperature. All reagents were brought to this temperature before the assay was actually performed.

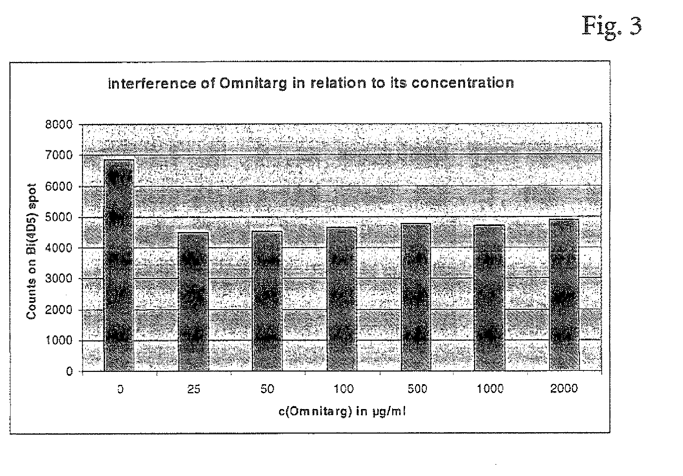

A sample containing 20 ng/ml of HER2 was incubated with various amounts of Omnitarg^{®} to result in the concentrations given in Fig. 3. These samples were diluted 1:5 in sample buffer. Doublets of 40 µl each of the diluted sample are added to the chips and incubated at RT for 10 min. Thereafter the chips are washed by an automated washing step. The conjugate antibody MAK<Her2>M-7C2-IgG-Dig was diluted to 3 µg/ml in conjugate buffer. 40 µl of diluted conjugate antibodies were added to each chip and incubated for 5 min followed by an automated washing step. The labeled detection antibody was diluted 1:5 in label (= detection) buffer. 40 µl of the diluted detection antibody are added to each chip and incubated for 5 min. After a final washing step the label bound is quantified using a laser with an excitation of 633 nm. A digital picture is converted into counts by an appropriate software. As can be seen from Fig. 3 Omnitarg^{®} (above a final concentration of 25 µg/ml) did interfere to a constant extend in the above assay.

### b) Measurement of HER2 in the presence of Omnitarg^{®}

For measurement of samples which might exhibit interference by Omnitarg^{®} the active ingredient of this drug (rhu Mab 2C4 G186 CP R9805AX) has been added to the sample buffer in a final concentration of 6.25 µg/ml (= sample buffer (A)).

For preparation of artificial samples the HER2 standard material was diluted to 500, 200, 100, 50, 20, 10, 5, 2.5, 1 and 0 ng/ml, respectively, in mouse serum. These samples were diluted 1:5 in sample buffer (A). Doublets of 40 µl each of the diluted sample are added to the chips and incubated at RT for 10 min. Thereafter the chips are washed by an automated washing step. The conjugate antibody MAK<Her2>M-7C2-IgG-Dig was diluted to 3 µg/ml in conjugate buffer. 40 µl of diluted conjugate antibodies were added to each chip and incubated for 5 min followed by an automated washing step. The labeled detection antibody was diluted 1:5 in detection buffer. 40 µl of the diluted detection antibody are added to each chip and incubated for 5 min. After a final washing step the label bound is quantified using a laser with an excitation of 633 nm. A digital picture is converted into counts by an appropriate software. The counts are given in Table 3.

**Table 3**

| **Counts obtained with MAK<Her2>H-4D5-IgG-Bi spots (calibration curve in sample buffer (A))** | |
|---|---|
| sHER2 in ng/ml | Counts on Bi(4D5) |
| 0 | 4 |
| 1 | 205 |
| 2.5 | 503 |
| 5 | 1064 |
| 10 | 3197 |
| 20 | 5201 |
| 50 | 16316 |
| 100 | 25107 |
| 200 | 38368 |
| 500 | 50449 |

As can be seen from Table 3, it was easily possible to establish a calibration curve in the presence of a basic level of Omnitarg^{®}.

### Example 3

### Assay for detection of HER2 in the presence of Herceptin^{®})

The assay was performed on streptavidin coated polystyrene chips. The antibody to HER2 was applied to the chip as lines of approximately ten 250 pL droplets. MAK<Her2>H-2C4-IgG-Bi (=biotmylated monoclonal antibody from clone 2C4 against HER2) was used to establish an assay devoid of Herceptin^{®} interference. The concentration of the biotinylated antibodies was 100 µg/ml The chips were stored at 4°C.

MAK<Her2>M-7C2-IgG-Dig (digoxigenylated monoclonal antibody from clone 7C2) was used as conjugate antibody. The stock solution of this conjugate was stored at -20°C.

The HER2 standard protein sp185 (HER-2) (sHER2), was a commercially available product (Biozol #BMS207MST S), and has been stored at -20°C in a concentration of 1000 ng/ml.

As the basic sample and conjugate buffer a phosphate buffered saline (50mM sodium dihydrogenphosphate-monohydrate, 150mM NaCl at pH 7), comprising sodium azide (0.09% Na-azide) as a preservative, and further additives (0.035% EDTA, 0.05% Tween 20^{®}, 2.00% RPLA4 (Roche Nr. 1726544001), 0.10% PAK<->R-IgG (Roche Nr. 1108750001), 100µg/ml mouse-KAK-33-IgG-Poly (Roche Nr. 1939661001)) was used. The sample and conjugate buffer has been filtered (0.2µm pore size) and stored at 4°C prior to use.

The stock solution of the therapeutic antibody Herceptin human variant Mab4D5 - produced by Roche Diagnostics GmbH - had a concentration of 25 mg/ml and was stored at -20°C.

As detection antibody MAK<Dig>MI9-11-IgG conjugated to 110 nm fluorescent labeled latex particles has been used. The detection antibody conjugate has been stored at 4°C.

As the detection buffer a 50 mM TAPS and 1M NaCl buffer at pH 8.5, comprising sodium azide (0.09% Na-azide) as a preservative, and further additives (0.05% Tween 20^{®}, 0.50% RPLA4 (Roche Nr. 1726544001), 10µg/ml mouse-MAK-33-IgG-Poly (Roche Nr. 1939661001)) was used. The detection buffer has been filtered (0.2µm pore size) and stored at 4°C prior to use.

The washing buffer was a 10mM Tris/HCl buffer with pH 8.0 comprising 0.001% Oxypyrion, 0.001% MIT, and 0.01% Thesit. The washing buffer has been filtered (0.2µm pore size) and stored at 4°C prior to use.

### a) Detection of a potential interference by Herceptin^{®}

The assay was performed at room temperature. All reagents were brought to this temperature before the assay was actually performed.

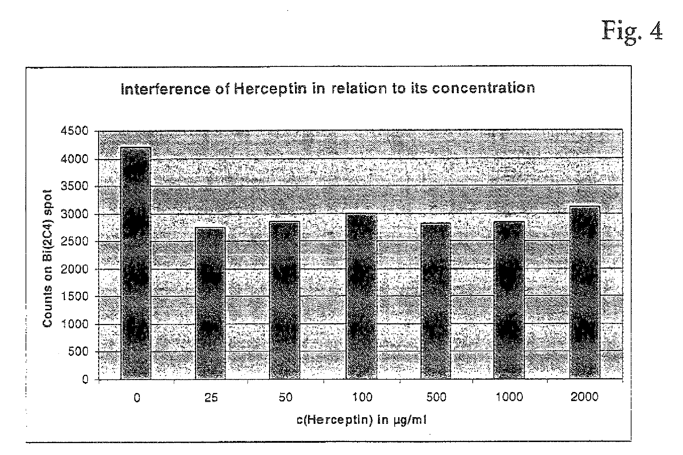

A sample containing 20 ng/ml of HER2 was incubated with various amounts of Herceptin^{®} to result in the concentrations given in Fig. 4. These samples were diluted 1:5 in sample buffer. Doublets of 40 µl each of the diluted sample are added to the chips and incubated at RT for 10 min. Thereafter the chips are washed by an automated washing step. The conjugate antibody MAK<Her2>M-7C2-IgG-Dig was diluted to 3 µg/ml in conjugate buffer. 40 µl of diluted conjugate antibodies were added to each chip and incubated for 5 min followed by an automated washing step. The labeled detection antibody was diluted 1:5 in detection buffer. 40 µl of the diluted detection antibody are added to each chip and incubated for 5 min. After a final washing step the label bound is quantified using a laser with an excitation of 633 nm. A digital picture is converted into counts by an appropriate software. As can be seen from Fig. 4 Herceptin^{®} (above a concentration of 25 µg/ml) did interfere to a constant extend in the above assay.

### b) Measurement of HER2 in the presence of Herceptin^{®}

For measurement of samples which might exhibit interference by Herceptin^{®} the active ingredient of this drug (MAK<Herceptin> 7.4.04) has been added to the sample buffer in a final concentration of 6.25 µg/ml (= sample buffer (B)). The sample buffer (B) has been filtered (0.2µm pore size) and stored at 4°C prior to use.

For preparation of artificial samples the HER2 standard material was diluted to 500, 200, 100, 50, 20, 10, 5, 2.5, 1 and 0 ng/ml, respectively, in mouse serum. These samples were diluted 1:5 in sample buffer (B). Doublets of 40 µl each of the diluted sample are added to the chips and incubated at RT for 10 min. Thereafter the chips are washed by an automated washing step. The conjugate antibody MAK<Her2>Mw7C2-IgG-Dig was diluted to 3 µg/ml in conjugate buffer. 40 µl of diluted conjugate antibodies were added to each chip and incubated for 5 min followed by an automated washing step. The labeled detection antibody was diluted 1:5 in detection buffer. 40 µl of the diluted detection antibody are added to each chip and incubated for 5 min. After a final washing step the label bound is quantified using a laser with an excitation of 633 nm. A digital picture is converted into counts by an appropriate software. The counts are given in Table 4.

**Table 4**

| **Counts obtained with MAK<Her2>H-2C4-IgG-Bi spots (calibration curve in sample buffer (B))** | |
|---|---|
| sHER2 in ng/ml | Counts on Bi(2C4) |
| 0 | 4 |
| 1 | 116 |
| 2.5 | 286 |
| 5 | 788 |
| 10 | 1975 |
| 20 | 3522 |
| 50 | 11541 |
| 100 | 18984 |
| 200 | 33293 |
| 500 | 48428 |

As can be seen from Table 4, it was easily possible to establish a calibration curve for HER2 in the presence of a basic level of Herceptin^{®}.

### Example 4

### Measurement of HER2 in a sample with and without therapeutic antibody, respectively

A serum pool of mice carrying the tumor KPL-4 (which is known to produce HER2) was prepared and divided into four aliquots.

Aliquots 1 und 2 were diluted 1:5 in sample buffer (B) and (A), respectively (=non-treated samples).

To aliquot 3 additional Herceptin^{®} was added so that the final concentration was 2000µg/ml. Then it was diluted with sample buffer (B) to also result finally in a 1:5 dilution as aliquot 1 (= treated sample 3).

To aliquot 4 additional Omnitarg^{®} was added so that the end concentration was 2000µg/ml. Then it was diluted with sample buffer (A) to also result finally in a 1:5 dilution as aliquot 2 (= treated sample 4).

The samples were measured as described in example 2b) and 3b), respectively. The results are shown in Table 5,

**Table 5**

| **Measurement of a sample in the presence of high levels of therapeutic antibody** | | | | |
|---|---|---|---|---|
| **sample/ "treatment"** | **sample buffer** | **Counts on 2C4 spots** | **Counts on 4D5 spots** | **difference between "non-treated" (1) and (2), respectively, and "treated" sample (3) and (4), respectively** |
| 1 / no | B | 23613 | | 100% |
| 2 / no | A | | 22758 | 100% |
| 3/ Herceptin^{®} | B | 21383 | | 90% = -10% difference |
| 4 / Omnitarg^{®} | A | | 21238 | 93% = -7% difference |

Despite the tremendous amounts of therapeutic antibody added, the differences between the non-treated and treated samples are not greater than 10%. Thus for both drugs Omnitarg^{®} as well as Herceptin^{®} an assay could be established which works even in the presence of high amounts of the corresponding therapeutic antibody.

## Claims

1. A method of detecting the target antigen of a therapeutic antibody in a sample comprising the steps of
a) providing the sample to be analyzed,
b) incubating said sample with said therapeutic antibody under conditions appropriate for binding of said therapeutic antibody to said target antigen, whereby a target antigen-therapeutic antibody-complex is formed, and
c) detecting the complex formed in (b).

2. The method according to claim 1, wherein said detecting is performed by way of an immuno assay.

3. The method according to claim 2, further **characterized in that** said immuno assay is a sandwich immuno assay.

4. The method according to one of claims 1 to 3, further **characterized in that** said therapeutic antibody is a human or a humanized antibody.

5. The method according to claim 4, further **characterized in that** said human or humanized antibody is a monoclonal antibody.

6. The method according to one of claims 1 to 5, wherein said therapeutic antibody is selected from the group consisting of Avastin^{®}, Herceptin^{®}, NiabTilera^{®}; Omnitarg^{®}, and Zenapax^{®}.

7. The method according to one of claims 1 to 5, wherein said target antigen is selected from the group consisting of HER2, CD20, and interleukin-2 receptor.

8. A method of detecting the target antigen of a therapeutic antibody in a sample wherein said therapeutic antibody is present comprising the steps of
a) providing the sample to be analyzed,
b) incubating said sample with said therapeutic antibody under conditions appropriate for binding of said therapeutic antibody to said target antigen, whereby a target antigen-therapeutic antibody-complex is formed, and
c) detecting the complex formed in (b).

9. A method of detecting the target antigen of a therapeutic antibody in a sample obtained from a patient under therapy with said therapeutic antibody comprising the steps of
a) providing the sample to be analyzed,
b) incubating said sample with said therapeutic antibody under conditions appropriate for binding of said therapeutic antibody to said target antigen, whereby a target antigen-therapeutic antibody-complex is formed, and
c) detecting the complex formed in (b).

10. Use of a method according to any of the preceding claims in the follow-up of patients.
